# DEMANDE DE BREVET EUROPEEN

(11) **EP 0 609 122 A1**
(43) Date de publication de la demande: **03.08.1994**
(21) Numéro de dépôt: 94400135.3
(22) Date de dépôt: 24.01.1994
(51) Int. Cl.: C07C 51/25, B01J 23/14, B01J 37/02, B01J 37/03

(54) **Composition de matière à base d'étain, catalyseurs à base de ces compositions dematière et leur utilisation pour la préparation d'acides carboxyliques insatures**

(30) Priorité: 29.01.1993 FR 9300938
(71) Demandeur: RHONE-POULENC CHIMIE, 92408 Courbevoie Cédex (FR)
(72) Inventeur: Blanchard, Gilbert, F-60330 Le Plessis Belleville (FR); Ferre, Gilbert, F-93190 Livry Gargan (FR)
(74) Mandataire: Dubruc, Philippe

(57) **Abrégé**

La présente invention a trait à des compositions de matières à base d'étain notamment et susceptible d'être obtenue en effectuant les étapes suivantes:
- on prépare une solution des éléments constitutifs de ladite composition;
- on précipite éventuellement lesdits éléments;
- on sèche la solution éventuellement traitée ;
- on calcine le produit séché.

Elle concerne de même des catalyseurs comprenant les compositions de matières précitées.

Elle concerne aussi l'utilisation desdits catalyseurs pour la préparation d'acides carboxyliques insaturés à partir de I'alcane correspondant, en présence d'une source d'oxygène.

## Description

La présente invention a trait à des compositions de matières à base d'étain notamment, ainsi qu'à leur mode de préparation. Elle concerne de même des catalyseurs comprenant les compositions de matières précitées ainsi que l'utilisation desdits catalyseurs pour la préparation d'acides carboxyliques insaturés.

De nombreux procédés concernant la préparation d'acides carboxyliques sont connus. De façon générale, les procédés consistent à faire réagir, en une ou plusieurs étapes, un alcane avec de l'oxygène pur ou dilué, en présence d'un catalyseur, pour donner, entre autres composés, l'acide carboxylique correspondant.

On connaît ainsi le brevet EP 117 146, décrivant l'oxydation du propane en acide acrylique en trois étapes successives. Ce procédé consiste tout d'abord, à déshydrogéner le propane en propylène, puis à oxyder ce composé en acroléine et enfin oxyder celui-ci en acide acrylique. Contrairement aux procédés décrits antérieurement, celui-ci a montré qu'il n'était pas nécessaire de séparer le propylène produit intermédiairement, des composés n'ayant pas réagi. Cependant, I'inconvénient majeur de ce procédé est qu'il est réalisé avec un catalyseur différent pour chacune des trois étapes. En outre, les catalyseurs mis en oeuvre sont complexes et/ou coûteux. En effet, le catalyseur de déshydrogénation cité comprend un métal noble, comme le platine, et les catalyseurs d'oxydation ne comprennent pas moins de 5 éléments différents. Or la tendance actuelle est de disposer, d'une part de catalyseurs uniques permettant d'obtenir directement et d'une façon la plus sélective possible, le produit désiré, et d'autre part de catalyseurs dont les compositions sont les plus simples possibles et les moins coûteuses.

Un autre procédé d'oxydation de propane en acide acrylique, décrit dans le brevet US 3 293 290, consiste à faire réagir un alcane avec de l'oxygène ou de l'air, en présence d'un catalyseur à base de phosphore et de molybdène d'une part, et d'éléments comme le bismuth et le fer d'autre part. Le point essentiel de ce procédé est qu'il est mis en oeuvre en présence d'un promoteur choisi parmi les acides bromhydrique ou iodhydrique.
Les performances d'un tel procédé ne sont pas, très satisfaisantes car la sélectivité en acroléine est pratiquement aussi importante que celle de l'acide acrylique. De plus, la réaction coproduit entre 40 et 60 % de monoxyde et dioxyde de carbone.
Enfin, l'emploi de promoteurs du type des acides mentionnés n'est pas souhaitée car leur pouvoir corrosif très important implique des conditions de mise en oeuvre très complexes à l'échelle industrielle.

On connaît par le brevet US 4 260 822, un procédé d'oxydation directe du propane en acide acrylique mettant en oeuvre un catalyseur à base d'un oxyde de molybdène, d'antimoine et de phosphore. Cependant, la sélectivité en acide acrylique obtenu dans une telle réaction, est de 19 % seulement.

Un autre procédé d'oxydation du propane en acide acrylique a de même été étudié en mettant en oeuvre un catalyseur à base d'oxyde de phosphore et de vanadium ("Oxydation of propane to acrylic acid" Catalysis today, 13, (1992) 679-684). La sélectivité maximale en acide acrylique est de 33 %.
L'inconvénient majeur de ce procédé est qu'il est mis en oeuvre avec des mélanges gazeux dont la composition est dans le domaine d'explosivité. Cet inconvénient est d'autant plus critique que la réaction d'oxydation est effectuée avec un catalyseur en lit fixe.

Comme on peut le constater, et ceci malgré de nombreuses recherches effectuées dans le domaine de l'oxydation catalytique directe d'alcanes en acide carboxyliques insaturés, on ne dispose toujours pas, à ce jour, de catalyseurs dont la sélectivité en acide soit suffisamment importante pour industrialiser ce type de procédé.

L'un des buts de la présente invention est donc de pallier ce manque et de décrire une composition de matière à base d'étain ainsi qu'un catalyseur dont la phase active est du type de la composition précitée.

Un autre but de la présente invention est de décrire un procédé d'obtention d'acides carboxyliques insaturés à partir d'alcane, mettant en oeuvre un catalyseur du type précité, et dont la sélectivité en acide formé est considérablement augmentée par rapport à celles des procédés connus.

Un autre but de l'invention est de décrire un procédé d'oxydation directe d'alcane en acide carboxylique dans lequel la coproduction en monoxyde et dioxyde de carbone est minimisée.

L'invention a pour autre objectif de décrire un procédé mettant en oeuvre des mélanges gazeux dont la composition est avantageusement en dehors du domaine d'explosivité desdits mélanges.

Ces buts et d'autres sont atteints par la présente invention qui concerne donc, tout d'abord, une composition de matière à base d'étain, d'oxygène et d'au moins un élément choisi parmi le vanadium, le niobium, le tantale, le chrome, le molybdène, le tungstène, l'aluminium, le gallium, l'indium, le silicium, le germanium, l'arsenic, le bismuth, le sélénium et le tellure, et susceptible d'être obtenue en effectuant les étapes suivantes :
- on prépare une solution des éléments constitutifs de ladite composition ;
- on précipite éventuellement lesdits éléments ;
- on sèche la solution éventuellement traitée ;
- on calcine le produit séché.

La présente invention concerne par ailleurs un catalyseur dont la phase active est du type de ladite composition de matière.

Elle concerne de même un procédé de préparation d'acides carboxyliques insaturés mettant en oeuvre ledit catalyseur en présence d'un mélange gazeux comprenant un alcane et une source d'oxygène.

Enfin, la présente invention décrit un procédé de préparation d'acides carboxyliques insaturés, en phase gaz, mettant en oeuvre un catalyseur dont la phase active est à base d'étain, d'oxygène et d'au moins un élément choisi parmi le vanadium, le niobium, le tantale, le chrome, le molybdène, le tungstène, l'aluminium, le gallium, l'indium, le silicium, le germanium, le phosphore, l'arsenic, l'antimoine, le bismuth, le sélénium et le tellure ; ladite phase active étant susceptible d'être obtenue en effectuant les étapes suivantes :
- on prépare une solution des éléments constitutifs de ladite composition ;
- on précipite éventuellement lesdits éléments ;
- on sèche la solution éventuellement traitée ;
- on calcine le produit séché.

Il a été trouvé de façon totalement surprenante que l'utilisation de catalyseurs à base d'étain tels que définis précédemment permet d'améliorer considérablement la sélectivité en acide carboxylique formé. En effet, dans le cas particulier de l'acide préparé à partir du propane, la sélectivité est d'au moins 40 % et avantageusement d'au moins 50 %.

Mais tous les avantages et caractéristiques du procédé selon l'invention apparaîtront plus clairement à la lecture de la description et des exemples qui vont suivre, auxquels sont annexées les figures suivantes :
figure 1 : diagramme ternaire propane - air - eau, indiquant les zones d'explosivité du mélange ;
figure 2 : diagramme ternaire propane - oxygène - eau et diluant, montrant la zone d'explosivité du mélange.

Ainsi qu'il a été dit plus haut, la composition de matière selon l'invention est caractérisée tout d'abord par les éléments la constituant et d'autre part, par son mode de préparation. Pour plus de commodité, les éléments constitutifs de ladite phase vont tout d'abord être décrits.

La composition selon l'invention est donc à base d'étain, d'oxygène et d'au moins un élément choisi parmi le vanadium, le niobium, le tantale, le chrome, le molybdène, le tungstène, l'aluminium, le gallium, l'indium, le silicium, le germanium, l'arsenic, le bismuth, le sélénium et le tellure.

Par ailleurs, ladite composition est telle que le rapport molaire de l'étain à la somme des éléments précités est compris entre 0,1 et 10 et de préférence compris entre 0,5 et 2.

Selon un mode de réalisation particulier de l'invention, la composition selon l'invention comprend de l'étain, de l'oxygène et au moins un élément choisi parmi le vanadium, le niobium, le chrome, le molybdène, le tungstène, le gallium et le bismuth.

Outre ses éléments constitutifs, ladite composition de matière est caractérisée par son mode de préparation.

Une première variante consiste à effectuer les étapes suivantes :
- on prépare une solution des éléments constitutifs de ladite composition ;
- on sèche la solution précitée ;
- on calcine le produit séché.

Selon une caractéristique particulière de l'invention, la solution précitée est à base de tous les éléments constitutifs de la composition de matière selon l'invention.

Lesdits éléments sont habituellement utilisés sous forme de sels d'acides inorganiques ou organiques, ou encore sous forme de composés comme les oxydes ou leurs dérivés.

Tous les acides ou composés indiqués conviennent à la préparation de la solution dans la mesure où le sel peut se décomposer en oxyde de l'élément correspondant.

A titre d'exemples de sels d'acides inorganiques convenant à la préparation de la solution précitée, on peut mentionner entre autres les nitrates, les sulfates, les halogénures comprenant un ou plusieurs halogènes, les sels d'ammonium.

A titre d'exemples de sels d'acides ou d'esters organiques, on peut mentionner le formiate, l'oxalate, le tartrate, I'acétate, I'acétylacétonate, l'éthylhexanoate.

Ainsi qu'il a été indiqué auparavant, il est aussi envisageable d'utiliser des oxydes ou leurs dérivés ; ces composés étant solubilisés, si nécessaire, par ajout d'un acide ou d'une base au mélange.
Par dérivés d'oxydes, on entend les composés du type des oxyhalogénures, des alkoxydes, les aryloxydes, les glycoxydes notamment.

Il est à noter que tous ces composés peuvent être utilisés seuls ou en mélange.

Sans intention de se limiter à des composés particuliers, les sels d'étain suivants peuvent être utilisés pour l'obtention de ladite solution : les sulfates d'étain, les chlorures d'étain, les bromures d'étain, les fluorures d'étain, les bromochlorures d'étain, les chloroiodures d'étain, I'acétate d'étain, l'oxalate d'étain, le tartrate d'étain, le 2-éthylhexanoate d'étain, le monoxyde d'étain, le dioxyde d'étain, I'éthoxyde d'étain, le méthoxyde d'étain, I'éthylèneglycoxyde d'étain.

A titre d'exemple de composés à base de vanadium, on peut citer notamment le sulfate de vanadyle, le vanadate d'ammonium, les oxyhalogénures de vanadium tels que, notamment VOCl₃, (VO₂)Cl, VOCI, VOBr, VOBr₂, VOF₃, VOF₂, VF₄, VBr₂, Vl₂, I'acétylacétonate de vanadyle, l'oxalate de vanadyle, l'acide métavanadique, l'hexacarbonyle de vanadium, le tri-isopropoxyde d'oxyde de vanadium, les oxydes de vanadium comme par exemple, V₂O₅, V₇O₁₃, VO, VO₂, V₂O₃, V₃O₇.

Parmi les composés du niobium utilisables on peut citer, toujours sans intention de se limiter, les chlorures de niobium, l'oxalate de niobium, l'oxychlorure de niobium, l'oxybromure de niobium, I'éthoxyde de niobium, le phénoxyde de niobium, les oxydes de niobium, comme par exemple Nb₂O₃, Nb₂O₄, Nb₂O₅.

En tant que composés à base de chrome, on peut mentionner les nitrates, les sulfates, les chlorures, les bromures, le perchlorate de chrome, les oxydes comme Cr₂O₃, les hydroxydes, l'hexacarbonyle de chrome, I'acétate, l'oxalate de chrome, le tartrate de chrome, I'acétylacétonate de chrome, le 2-éthylhexanoate de chrome.

A titre d'exemple de composés apportant le molybdène, ou le tungstène, on peut mentionner notamment le dimolybdate d'ammonium, l'heptamolybdate d'ammonium, le métatungstate d'ammonium, le paratungstate d'ammonium.

Les composés apportant le gallium peuvent être choisis parmi les nitrates, les sulfates, les chlorures, les bromures, l'oxychlorure, le perchlorate de gallium, les oxydes de gallium comme notamment Ga₂O₃, I'acétate de gallium, l'oxalate de gallium, I'acétylacétonate de gallium.

Les concentrations en chacun des éléments mis en solution est ajustée de telle sorte que le rapport molaire étain sur la somme des autres éléments soit comprise entre 0,1 et 10 et de préférence entre 0,5 et 2.

La solution précitée est donc obtenue par dissolution des sels des éléments constitutifs de la phase active dans tout solvant adéquat.

De façon avantageuse, on effectue la solubilisation des éléments en présence d'eau. Il n'est cependant pas exclu d'utiliser tout autre solvant organique, dans la mesure où il solubilise bien lesdits sels comme certains alcools tels que l'isopropanol, le tertiobutanol.

Généralement, la préparation de la solution est faite sous agitation.

La température à laquelle on solubilise les éléments constitutifs est habituellement comprise entre 20°C et la température d'ébullition du solvant choisi.

De façon générale, la concentration de la solution varie entre 0,1 et 5 M.

Une fois la solution obtenue, on procède au séchage de celle-ci.

Selon un premier mode de réalisation, le séchage est effectué en deux étapes: la première consistant à évaporer le solvant du mélange, jusqu'à siccité, la seconde à sécher la pâte ainsi obtenue.

Généralement, la première étape est réalisée à une température variant de 20 à 100°C pendant la durée nécessaire pour obtenir une pâte non coulante.

L'évaporation est habituellement effectuée sous agitation.

La pâte résultante est ensuite séchée, dans une seconde étape, sous une atmosphère de préférence non réductrice, comme l'oxygène ou l'air par exemple, pendant une durée moyenne de 15 heures.

La température de séchage est habituellement d'environ 120°C.

Dans un second mode de réalisation, le séchage est effectué en atomisant la solution, par tout moyen connu de l'homme du métier. Ainsi, sans intention de se limiter, les atomiseurs de type BUCHI ou encore les atomiseurs de type "flash" comme revendiqués dans les demandes de brevets français publiées sous les numéros suivants : 2 257 326, 2 419 754, 2 431 321, conviennent à ce mode de réalisation.

La température d'atomisation est généralement de l'ordre de 150 à 300°C.

L'atmosphère sous laquelle est effectuée l'atomisation, là encore, est de préférence non réductrice. De façon avantageuse, on effectue l'atomisation sous air, bien que l'oxygène soit envisageable pour une telle étape.

Le produit séché obtenu selon chacun des deux modes de réalisation précités, est ensuite soumis à une étape de calcination.

Celle-ci est effectuée, de façon classique, sous une atmosphère non réductrice. D'une façon avantageuse celle-ci est effectuée sous air, mais elle pourrait cependant être réalisée sous oxygène.

La température de calcination est habituellement comprise entre 200 et 1200°C.

Généralement, la durée de l'opération varie entre 1 et 24 heures.

Préalablement à l'étape de calcination, le produit séché peut subir une étape de broyage.

Il est à noter que le produit calciné peut éventuellement subir aussi une telle étape.

Une seconde variante de préparation de la composition selon l'invention consiste à effectuer les étapes suivantes :
- on prépare une solution des éléments constitutifs de ladite composition ;
- on précipite lesdits éléments ;
- on sèche le mélange obtenu ;
- on calcine le produit séché.

La principale caractéristique différenciant ce mode de réalisation du premier, est qu'il comporte une étape de précipitation intermédiaire des éléments. Par conséquent, tout ce qui a été décrit auparavant, concernant notamment la préparation de la solution des éléments constitutifs de la composition selon l'invention, les étapes de séchage, de calcination, éventuellement de broyage, reste bien entendu valable dans la cadre de cette seconde variante et ne sera pas repris dans cette partie.

Selon une caractéristique particulière de l'invention, on effectue une précipitation simultanée des éléments constitutifs de la composition selon l'invention.

Tout type d'agent précipitant convient à la mise en oeuvre de cette variante dans la mesure où il se combine à un ou plusieurs éléments constitutifs sous la forme d'un composé insoluble dans le milieu choisi et dans la mesure où ledit composé insoluble permet d'accéder à l'oxyde du ou des éléments combinés.

Il est à noter que la précipitation peut être réalisée avec un ou plusieurs agents précipitants.

Classiquement, ledit agent est choisi, entre autres parmi les bases du type de l'ammoniaque notamment, ou parmi les acides inorganiques ou organiques, comme l'acide chlorhydrique ou encore l'acide citrique. Bien évidemment le choix des agents précipitants n'est pas limité à cette seule liste, donnée à titre indicatif.

Habituellement, la précipitation est mise en oeuvre sous agitation.

La température à laquelle elle est réalisée varie généralement entre 20°C et la température d'ébullition du solvant choisi.

Enfin, I'étape de précipitation peut si nécessaire être suivie d'une étape de mûrissement. Celle-ci consiste à laisser le précipité en suspension, sous agitation, pendant une durée variant de 30 minutes et 4 heures environ.

La température de mûrissement est comprise dans la gamme indiquée précédemment.

Enfin, le mélange obtenu peut être filtré avant les opérations de séchage et de calcination telles que décrites auparavant.

Un second objet de l'invention est constituée par un procédé de préparation de la composition catalytique décrite ci-dessus.

Ce procédé comporte deux modes particuliers dont le premier consiste à effectuer les étapes suivantes :
- on prépare une solution des éléments constitutifs de ladite composition ;
- on sèche la solution obtenue ;
- on calcine le produit séché.

Le second mode particulier de préparation de la composition selon l'invention consiste à effectuer les étapes suivantes :
- on prépare une solution des éléments constitutifs de ladite composition ;
- on précipite lesdits éléments ;
- on sèche le mélange obtenu ;
- on calcine le produit séché.

Tout ce qui a été décrit auparavant concernant la préparation de ladite composition, en tant que caractéristique de produit, reste bien entendu valable et ne sera pas repris en détails dans cette partie de la description. Il suffira donc de s'y reporter.

Un troisième objet de l'invention est constitué par un catalyseur dont la phase active est à base de la composition de matière telle que précédemment décrite.

Selon un mode de réalisation particulier de l'invention, la phase active dudit catalyseur est essentiellement constituée par la composition de matière précitée.

Par conséquent, et pour tout le reste de la description, on entend par phase active, la composition de matière selon l'invention, et par précurseur de la phase active, la composition selon l'invention, sous la forme des sels de ses éléments constitutifs ; ces derniers étant ou non séchés.

Le catalyseur selon l'invention peut se présenter indifféremment sous forme de monolithe ou de particules.

Dans le cas où le catalyseur est constitué de particules, la granulométrie de celles-ci dépend du mode d'utilisation du catalyseur. Elle peut donc varier dans de larges limites, comme notamment être comprise entre quelques micromètres et une dizaine de millimètres. Plus particulièrement, et à titre indicatif, un catalyseur utilisé en lit fixe, présente une répartition granulométrique généralement comprise entre 0,5 et 6 mm. La granulométrie des particules d'un catalyseur utilisé en lit fluidisé ou mobile, est habituellement comprise entre 5 et 700 microns, et de préférence comprise entre 5 et 200 microns pour 80 % des particules.

Par ailleurs, le catalyseur selon l'invention peut se présenter sous forme massique ou supportée.

Dans le cas particulier où le catalyseur comprend un support, la phase active peut être soit déposée sur celui-ci, soit l'enrober ou encore y être mélangée.

La nature du support n'est pas critique, si ce n'est qu'il doit être inerte vis-à-vis des réactifs dans les conditions réactionnelles choisies.

A titres de matières susceptibles d'être utilisés comme support de catalyseur, on peut citer : la silice, l'alumine, la silice-alumine, l'argile frittée, la magnésie, le silicate de magnésium, la terre de diatomée. Ces types de support peuvent être utilisés de préférence sous forme non poreuse. Si nécessaire, on peut effectuer un émaillage de ceux-ci afin de les rendre tels.

Les matériaux céramiques, du type de la cordiérite, l'alumine, la mullite, la porcelaine, le nitrure de silicium, les carbures de bore et de silicium, peuvent aussi être utilisés comme support de catalyseur.

Ce type de matériaux convient plus particulièrement à la réalisation de catalyseur sous forme monolithique comprenant des canaux.

D'une façon classique, la quantité de support entrant dans la composition d'un catalyseur varie dans de larges limites. Ainsi, les catalyseurs obtenus par enrobage ou dépôt de la phase active sur le support présentent une quantité de phase active variant habituellement entre 1 et 50 % et de préférence entre 5 et 35 % en poids total dudit catalyseur (phase active et support). Dans les cas où le catalyseur est constitué d'un support dispersé dans la phase active, la quantité de phase active est comprise entre 10 et 90 % en poids total de catalyseur.

Le catalyseur selon l'invention peut être obtenu en mettant en oeuvre des méthodes classiques.

Ainsi, les catalyseurs massiques, comprenant essentiellement la phase active telle que définie plus haut, peuvent être mis en forme par extrusion, par moulage, par broyage, concassage ou tout autre moyen, de façon à donner un monolithe ou encore des particules de granulométrie convenable.

Dans le cas de catalyseurs comprenant un support, plusieurs méthodes sont envisageables.

Selon un premier mode de réalisation, on met en contact le support, de préférence sous forme de particules rugueuses, et la phase active ou son précurseur, dans un mélangeur à fort cisaillement (appareils type LODIGE) ou dans un appareil de granulation (drageoirs, sous forme de tambour ou assiette).

L'opération est effectuée en général à une température variant entre 20 et 150°C pendant la durée nécessaire à l'enrobage du support par la quantité désirée de phase active, généralement sous air, pendant au moins 30 minutes.

Les particules ainsi obtenues peuvent ensuite être calcinées à une température comprise entre 300 et 600°C, de préférence entre 450 et 500°C. La durée du séchage est d'au moins 3 heures.

Un second mode possible de fabrication du catalyseur consiste à mettre en application la technique d'imprégnation.

Selon cette technique, on imprègne le support avec une suspension de la phase active ou de son précurseur. On peut de même imprégner ledit support avec une solution des éléments constitutifs de la phase active.

L'étape d'imprégnation est suivie d'une étape de séchage, habituellement effectuée à une température comprise entre 100 et 200°C, sous air, pendant au moins 30 minutes.

On peut ensuite renouveler le cycle imprégnation - séchage et terminer celui-ci par une calcination sous air, à une température comprise entre 400 et 600°C, pendant une dizaine d'heures. Une variante possible consiste à effectuer une calcination entre le ou les cycles imprégnation-séchage.

Selon un troisième mode de préparation du catalyseur, on prépare un mélange comprenant la solution des éléments constitutifs de la composition selon l'invention et le support, se présentant plus particulièrement sous forme de particules. Le mélange ainsi obtenu est ensuite traité conformément aux deux variantes du procédé de préparation de la composition selon l'invention. De préférence, on met en oeuvre la première variante, c'est-à-dire celle consistant à sécher le mélange sans précipitation intermédiaire des éléments constitutifs.

Cette méthode est particulièrement intéressante dans la mesure où elle permet, d'obtenir un catalyseur de type supporté enrobé.

Bien entendu, tous ces modes de préparation ne sont donnés qu'à titre indicatif et ne peuvent en aucun cas constituer une liste exhaustive.

La présente invention concerne par ailleurs un procédé d'obtention d'acides carboxyliques insaturés consistant à mettre en contact un mélange gazeux, comprenant un alcane et une source d'oxygène, avec un catalyseur comme décrit précédemment. La présente invention concerne enfin un procédé d'obtention d'acides carboxyliques insaturés consistant à mettre en contact un mélange gazeux comprenant un alcane et une source d'oxygène, avec un catalyseur dont la phase active est à base d'étain, d'oxygène et d'au moins un élément choisi parmi le vanadium, le niobium, le tantale, le chrome, le molybdène, le tungstène, l'aluminium, le gallium, l'indium, le silicium, le germanium, le phosphore, l'arsenic, l'antimoine, le bismuth, le sélénium et le tellure ; ladite phase active étant susceptible d'être obtenue en effectuant les étapes suivantes :
- on prépare une solution des éléments constitutifs de ladite composition ;
- on précipite éventuellement lesdits éléments ;
- on sèche la solution éventuellement traitée ;
- on calcine le produit séché.

Ladite phase active présente une composition telle que le rapport molaire de l'étain à la somme des éléments précités est compris entre 0,1 et 10 et de préférence entre 0,5 et 2.

Selon un mode de réalisation particulier de l'invention, la phase active, outre l'étain et l'oxygène, comprend au moins un élément choisi parmi le vanadium, le niobium, le chrome, le molybdène, le tungstène, l'antimoine, le gallium, le bismuth.

Tout ce qui a été décrit auparavant concernant la préparation de la composition de matière selon l'invention est bien entendu applicable à l'obtention de la phase catalytique dudit catalyseur à base d'étain, de même que les diverses méthodes connues de préparation de catalyseurs, citées à titre d'exemples.

Le procédé d'obtention d'acides carboxyliques insaturés à partir d'un alcane et d'une source d'oxygène sur des catalyseurs à base d'étain est décrit ci-après.

Il a été trouvé de façon totalement surprenante et inattendue que l'utilisation de catalyseurs tels que décrits auparavant, permet d'obtenir l'acide carboxylique insaturé avec une sélectivité augmentée par rapport à celle obtenue par mise en oeuvre des procédés connus. Ceci présente l'avantage de simplifier l'étape de séparation ultérieure des produits finals des réactifs et/ou sous produits. L'une des conséquences de cette simplification est que le recyclage de l'alcane n'ayant pas réagi en est aussi facilité, rendant de ce fait le procédé industrialisable, même avec un taux de transformation dudit alcane de l'ordre d'une dizaine de pour-cent.

Le procédé de préparation d'acides carboxyliques correspond donc à la réaction d'un mélange gazeux comprenant un alcane et une source d'oxygène, en présence d'un catalyseur.

Selon un mode particulier de réalisation de l'invention, l'alcane précité est un composé acyclique, présentant en outre 3 ou 4 atomes de carbone.

De préférence, l'alcane est le propane.

Il n'y a pas de conditions particulières concernant la qualité de l'alcane mis en oeuvre. Cependant, pour des questions évidentes de séparation de l'acide formé, on préfère utiliser un alcane de pureté d'au moins 90 %.

Ce dernier peut être utilisé indifféremment sous forme pure ou diluée dans un gaz diluant, inerte dans les conditions de la réaction. Les gaz rares, comme notamment l'hélium ou l'argon, ou encore l'azote sont des gaz diluants convenant à la mise en oeuvre du procédé selon l'invention.

La réaction d'oxydation de l'alcane est de plus mise en oeuvre en présence d'une source d'oxygène. Celle-ci peut être à base d'oxygène pur ou dilué dans un gaz inerte. Ainsi on peut effectuer la réaction d'oxydation en utilisant de l'air comme source d'oxygène.

Selon un mode particulier de réalisation de l'invention, le mélange gazeux précité comprend en outre de l'eau.

Il a été remarqué de façon surprenante que la réaction d'oxydation permet d'obtenir des sélectivités en acide augmentées par rapport aux procédés connus, même si celle-ci est effectuée en l'absence d'eau.

La composition du mélange gazeux peut varier dans de larges limites. Il est rappelé que par mélange gazeux, on entend le mélange alcane / oxygène, comprenant éventuellement un gaz diluant et/ou de l'eau.

Sauf mention contraire, tous les pourcentages indiqués par la suite, sont exprimés en volume total du mélange gazeux.

D'une façon générale, la teneur en alcane dans le mélange gazeux, est comprise entre 10 et 70 %. Plus particulièrement, ladite teneur en alcane est d'au moins 20% et de préférence d'au moins 30 %.

La teneur en oxygène est aussi comprise dans une large gamme de teneur. Celle-ci varie habituellement entre 10 et 70 %.

La teneur en eau dans le gaz mis en oeuvre varie en général de 0 à 50 %. Selon un mode de réalisation particulier, la teneur en eau dans le mélange précité est comprise entre 0 et 30%.

La teneur en gaz diluant dans le mélange, varie habituellement entre 0 et 50%. Plus particulièrement le mélange comprend entre 0 et 30 % de gaz diluant.

Il est à noter, ainsi que cela a été mentionné auparavant, que la composition du mélange gazeux est telle qu'elle se trouve de préférence en dehors de la zone d'explosivité dudit mélange. Ceci peut notamment avoir des conséquences sur le rapport alcane/oxygène dans le mélange gazeux, selon le mode d'utilisation du catalyseur. Par exemple, dans le cas particulier d'une mise en oeuvre du catalyseur en lit fixe, la réaction peut être effectuée en présence d'un mélange gazeux dont le rapport alcane/oxygène est au moins égal à 1. Cependant, des rapports plus faibles ne sont pas exclus.

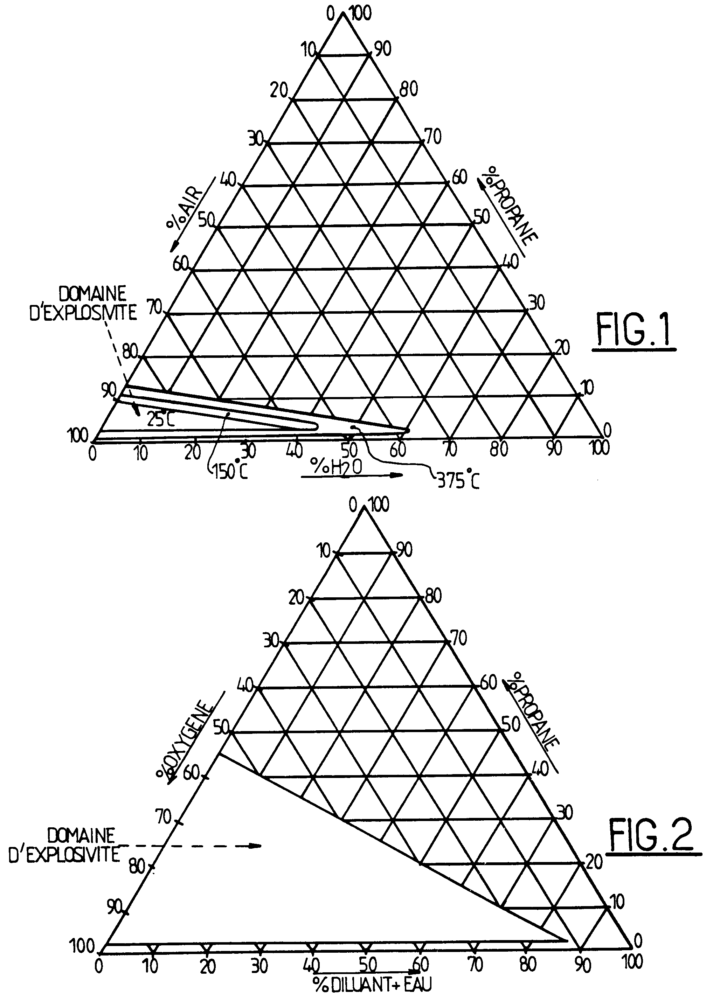

Dans le cas particulier de mise en oeuvre de l'invention avec le propane et de l'air comme source d'oxygène, la figure 1 montre qu'il existe trois domaines d'explosivité représentés par les surfaces non hachurées de la figure 1 ; ces domaines étant compris dans le triangle correspondant respectivement aux compositions suivantes :
(% propane - % air - % eau) (13/87/0) ; (2/36/62) ; (1/99/0).

Selon un second mode de réalisation particulier du procédé, où l'on met en oeuvre un mélange gazeux comprenant du propane, de l'oxygène, la figure 2 montre que le domaine d'explosivité à température ambiante correspond à la surface du triangle délimité par les points, dont les compositions sont les suivantes :
(% propane - % oxygène - % eau) (45/55/0) ; (1/11/88) ; (1/99/0).
Ces deux figures montrent clairement que les compositions de mélange gazeux mis en oeuvre dans le procédé selon l'invention ne sont avantageusement pas comprises dans les domaines indiqués ci-dessus.

Le mélange gazeux est donc mis au contact du catalyseur selon l'invention.

Le dispositif dans lequel le procédé selon l'invention est mis en oeuvre fait partie des dispositifs classiques pour les réactions catalytiques en phase gaz ; ces derniers pouvant être utilisés en continu ou en discontinu. Ainsi, on peut effectuer la réaction en présence d'un catalyseur en lit fixe, fluidisé ou encore transporté.

La température de réaction est en général située entre 250 et 600°C et de préférence entre 300 et 500°C.

La pression totale du mélange gazeux réactionnel peut être supérieure ou égale à la pression atmosphérique. Elle est généralement comprise entre 1 et 6 bar et plus particulièrement entre 1 et 4 bar.

Le débit gazeux est fixé de telle sorte que la vitesse volumique horaire soit comprise entre 100 et 10 000 h⁻¹, et de préférence entre 200 et 3600h⁻¹. Il est rappelé que la vitesse volumique horaire se définit comme le rapport volume gazeux total / volume de catalyseur / heure.

Bien évidemment, l'homme du métier est à même de trouver un compromis entre la température, le débit gazeux, la nature précise du catalyseur mis en oeuvre et les divers autres paramètres de la réaction, en fonction de ses objectifs de production.

L'acide carboxylique formé est séparé des sous produits ou des réactifs selon les méthodes classiquement utilisées.

Ainsi, une méthode connue consiste à refroidir le mélange réactionnel avec de l'eau ou tout solvant à haut point d'ébullition. L'acide carboxylique formé est ensuite extrait avec un solvant adéquat, comme notamment l'acétate d'éthyle dans le cas particulier de l'acide acrylique, puis séparé et purifié par distillation.

Des exemples concrets mais non limitatifs de l'invention vont maintenant être présentés.

### EXEMPLES

Dans tous les exemples, sauf indication expresse du contraire, les abréviations utilisées ont les significations suivantes :
- TT_{C3H8} =: taux de conversion du propane
= % propane transformé / % propane introduit
- S_{ACRY} =: sélectivité en acide acrylique
= % propane transformé en acide acrylique / % propane converti
- S_{C₃H₆} =: sélectivité en propylène
= % propane transformé en propylène / % propane converti
- S_{COx} =: sélectivité en monoxyde et dioxyde de carbone
= % propane transformé en monoxyde et dioxyde de carbone /% propane converti

### EXEMPLE 1:

### a) Préparation du catalyseur

On prépare : une solution (a) d'heptamolybdate d'ammonium par dissolution de 35,32g de (NH₄)₆Mo₇O₂₄,4H₂O dans 200 cm³ d'eau permutée,
une solution (b) de chlorure d'étain par dissolution de 45,13g de SnCl₂,2H₂O dans 200 cm³ d'eau permutée, acidifiée par quelques gouttes d'acide chlorhydrique concentré.

La solution (b) est ajoutée à la solution (a) dans un réacteur agité. La composition du mélange résultant est telle que le rapport atomique Sn/Mo est de 1/1.

On chauffe ledit mélange et l'on ajoute de l'ammoniaque jusqu'à un pH neutre.

On filtre sur verre fritté le précipité obtenu et on le lave avec 100 cm³ d'eau permutée.

Ledit précipité est ensuite séché à 120°C pendant 15h environ, broyé dans un mortier puis calciné sous air à 500°C pendant 3h.

On introduit 20g du produit calciné résultant dans 100 cm³ d'eau permutée, et l'on chauffe sous agitation à 70°C pendant 30 mn environ.

La suspension résultante est alors déposée sur 123g de support inerte composé de billes d'argile de diamètre moyen 4,8 mm.

On sèche ensuite à 120°C pendant 2h puis on calcine le produit séché à 480°C pendant 6h.

Le catalyseur résultant est constitué de 11 % en poids de phase active, déposée sur des billes d'argile.

### b) Mesure des performances du catalyseur en présence d'eau :

Le catalyseur précédemment préparé est testé pour l'oxydation du propane, dans les conditions opératoires suivantes :
- composition du mélange réactionnel
   65 % en volume de propane
   5 % en volume d'oxygène
   20 % en volume d'eau
   10 % en volume d'azote
- volume de catalyseur (phase active + billes d'argile) = 20 cm³
- débit total du mélange synthétique = 40 lh⁻¹
- vitesse volumique horaire (V.V.H.) = 2000 h⁻¹
- pression absolue : 1 bar
- température d'entrée des gaz dans le réacteur = 150 à 550°C incrémentée par paliers de 20°C.

La teneur des gaz en sortie de réacteur est déterminée par chromatographie en phase gazeuse.

Le tableau ci-après rassemble les résultats obtenus avec le catalyseur :

| T (°C) | TT_{C3H8} (%) | S_{ACRY} (%) | S_{C3H6} (%) | S_{COx} (%) |
|---|---|---|---|---|
| 340 | 1,5 | 82 | 0 | 8 |
| 360 | 2 | 76 | 6 | 3 |
| 380 | 3 | 56 | 17 | 15 |

### EXEMPLE 2:

Cet exemple concerne l'application du catalyseur décrit dans l'exemple 1 pour l'oxydation du propane en absence d'eau.

Ainsi, le catalyseur est mis en oeuvre dans les conditions opératoires suivantes :
- composition du mélange réactionnel
   50 % en volume de propane
   20 % en volume d'oxygène
   30 % en volume d'azote
- volume de catalyseur (phase active + billes d'argile) = 20 cm³
- débit total du mélange synthétique = 40 lh⁻¹
- vitesse volumique horaire (V.V.H.) = 2000 h⁻¹
- pression absolue : 1 bar.

Le tableau ci-après rassemble les résultats obtenus :

| T (°C) | TT_{C3H8} (%) | S_{ACRY} (%) | S_{C3H6} (%) | Scox(%) |
|---|---|---|---|---|
| 330 | 2 | 62 | 0 | 6 |
| 360 | 4 | 48 | 18 | 10 |

### EXEMPLE 3 COMPARATIF :

### a) Préparation d'un catalyseur de composition CeMoO_{X}

On prépare une solution (a) d'heptamolybdate d'ammonium de 52,98g de (NH₄)₆Mo₇O₂₄,4H₂O dans 200 cm³ d'eau permutée,
une solution (b) de nitrate de cérium par dissolution de 89,53g de Ce(NO₃)₃,6H₂O dans 200 cm³ d'eau permutée.

On ajoute la solution (b) à la solution (a) dans un réacteur fortement agité. La composition du mélange résultant est telle que le rapport atomique de Ce/Mo est de 2/3.

On évapore à 100-110°C et la pâte obtenue est séchée à 120°C pendant 15h environ.

Le produit obtenu est ensuite broyé dans un mortier puis calciné sous air à 550°C pendant 16h.

20g du produit ainsi obtenu sont déposés sur 123g de support inerte composé de billes d'argile de diamètre moyen 4,8mm.

On sèche ensuite à 120°C pendant 2h puis on calcine à 480°C pendant 6h.

Le catalyseur ainsi obtenu est constitué de 11,5 % en poids de phase active.

### b) Mesure des performances du catalyseur comparatif

Le catalyseur a été testé pour l'oxydation du propane dans les conditions de test mentionnées dans les exemples 1 et 2.

Les résultats obtenus avec ce catalyseur sont rassemblés dans le tableau ci-dessous :

| C₃H₈/O₂/H₂O/N₂ (%) | 65/5/20/10 | | | 50/20/0/30 | | |
|---|---|---|---|---|---|---|
| T (°C) | 450 | 480 | 500 | 450 | 480 | 500 |
| TT_{C3H8} (%) | 0 | 0,5 | 2 | 3 | 4 | 5 |
| S_{ACRY} (%) | / | 0 | 0 | 0 | 4 | 6 |
| S_{C3H6} (%) | / | 91 | 61 | 35 | 30 | 27 |
| Scox (%) | / | 9 | 7 | 8 | 7 | 7 |

On peut donc constater que ce catalyseur est beaucoup moins actif que le catalyseur de l'invention puisqu'il faut porter la température au moins jusqu'à 450°C pour convertir le propane, et qu'il est beaucoup moins sélectif en acide acrylique que le catalyseur de l'invention.

### EXEMPLE 4 COMPARATIF :

### a) Préparation d'un catalyseur de composition MoO₃-SnO₂

On mélange sous agitation, 64,8g de MoO₃ et 7,5g de SnO₂ dans 260 cm³ d'eau permutée.

On porte à ébullition pendant 2h30, on sèche le mélange obtenu à 120°C pendant 18h puis on calcine le produit séché sous air à 650°C pendant 6h.

Le produit obtenu a été ensuite déposé sur 50 g de support inerte composé de billes d'argile de diamètre moyen 4,8 mm.

On sèche ensuite à 120°C pendant 2h puis on calcine à 480°C pendant 6h.

Le catalyseur ainsi obtenu est constitué de 9,6 % en poids de phase active, correspondant à un rapport atomique Sn/Mo de 1/9.

### b) Mesure des performances du catalyseur comparatif.

Le catalyseur a été testé pour l'oxydation du propane dans les conditions de test mentionnées dans les exemples 1 et 2.

Les résultats obtenus avec ce catalyseur sont rassemblés dans le tableau ci-dessous :

| C₃H₈/O₂/H₂O/N₂ (%) | 65/5/20/10 | | | | 50/20/0/30 | | | |
|---|---|---|---|---|---|---|---|---|
| T(°C) | 375 | 400 | 450 | 475 | 375 | 400 | 450 | 500 |
| TT_{C3H8} (%) | 0 | 1,1 | 0,7 | 0,9 | 0,3 | 0,3 | 0,6 | 1,4 |
| S_{ACRY} (%) | / | 0 | 0 | 0 | 0 | 0 | 0 | 2 |
| S_{C3H6} (%) | / | 6 | 19 | 27 | 0 | 0 | 41 | 60 |
| Scox (%) | / | 0 | 0 | 0 | 0 | 0 | 11 | 6 |

## Revendications

**1/** Composition de matière à base d'étain, d'oxygène et d'au moins un élément choisi parmi le vanadium, le niobium, le tantale, le chrome, le molybdène, le tungstène, l'aluminium, le gallium, l'indium, le silicium, le germanium, l'arsenic, le bismuth, le sélénium et le tellure, et susceptible d'être obtenue en effectuant les étapes suivantes :
- on prépare une solution des éléments constitutifs de ladite composition ;
- on sèche la solution ;
- on calcine le produit séché.

**2/** Composition de matière à base d'étain, d'oxygène et d'au moins un élément choisi parmi le vanadium, le niobium, le tantale, le chrome, le molybdène, le tungstène, l'aluminium, le gallium, l'indium, le silicium, le germanium, l'arsenic, le bismuth, le sélénium et le tellure, et susceptible d'être obtenue en effectuant les étapes suivantes :
- on prépare une solution des éléments constitutifs de ladite composition ;
- on précipite lesdits éléments ;
- on sèche le mélange obtenu ;
- on calcine le produit séché.

**3/** Composition de matière selon l'une quelconque des revendications précédentes, caractérisée en ce qu'elle est à base d'étain, d'oxygène et d'au moins un élément choisi parmi le vanadium, le niobium, le chrome, le molybdène, le tungstène, le gallium et le bismuth.

**4/** Composition de matière selon l'une quelconque des revendications précédentes, caractérisée en ce que le rapport molaire de l'étain à la somme des éléments précités compris entre 0,1 et 10 et de préférence entre 0,5 et 2.

**5/** Composition de matière selon l'une quelconque des revendications précédentes, caractérisée en ce que la solution précitée est une solution aqueuse.

**6/** Composition de matière selon l'une quelconque des revendications précédentes, caractérisée en ce que les éléments constitutifs sont utilisés sous forme de sels solubles d'acides inorganiques, choisis parmi les nitrates, les sels d'ammonium, les sulfates, les chlorures, les bromures, les iodures, ou leurs mélanges.

**7/** Composition de matière selon l'une quelconque des revendications 1 à 5, caractérisée en ce que les éléments constitutifs sont utilisés sous forme de sels solubles d'acides organiques choisis parmi le formiate, l'oxalate, le tartrate, I'acétate, I'acétylacétonate, l'ethylhexanoate, ou leurs mélanges.

**8/** Composition de matière selon l'une quelconque des revendications 1 à 5, caractérisée en ce que les éléments constitutifs sont des composés solubles choisis parmi les oxydes, ou leurs dérivés comme les oxyhalogénures, les alkoxydes, les aryloxydes, les glycoxydes ; ces composés étant solubilisés si nécessaire par ajout d'un acide ou d'une base.

**9/** Composition de matière selon l'une quelconque des revendications 2 à 8, caractérisée en ce que l'on ajoute à la solution précitée, un agent précipitant choisi parmi les bases, comme l'ammoniaque, ou les acides inorganiques ou organiques, comme l'acide chlorhydrique, l'acide citrique.

**10/** Composition de matière selon la revendication précédente, caractérisée en ce que l'on soumet le mélange ainsi obtenu à une étape de mûrissement, sous agitation, pendant une durée comprise entre 30 minutes et 4 heures.

**11/** Composition de matière selon l'une quelconque des revendications précédentes, caractérisée en ce que l'on effectue le séchage dudit mélange, sous air, par évaporation.

**12/** Composition de matière selon l'une quelconque des revendications 1 à 10, caractérisée en ce que l'on effectue le séchage dudit mélange, sous air, par atomisation à une température comprise entre 150 et 300°C.

**13/** Composition de matière selon l'une quelconque des revendications précédentes, caractérisée en ce que l'on effectue une calcination du produit séché sous air.

**14/** Composition de matière selon la revendication précédente, caractérisée en ce que l'on effectue la calcination à une température comprise entre 200 et 1200°C pendant 1 à 24 heures.

**15/** Composition de matière selon l'une des revendications précédentes, caractérisée en ce que l'on effectue une étape de broyage préalablement et/ou postérieurement à la calcination.

**16/** Procédé de préparation de la composition de matière selon l'une quelconque des revendications 1, 3 à 8 et 11 à 15, caractérisé en ce que l'on effectue les étapes suivantes :
- on prépare une solution des éléments constitutifs de ladite composition ;
- on sèche la solution précitée ;
- on calcine le produit séché.

**17/** Procédé de préparation de la composition de matière selon l'une quelconque des revendications 2 à 15, caractérisé en ce que l'on effectue les étapes suivantes :
- on prépare une solution des éléments constitutifs de ladite composition ;
- on précipite lesdits éléments ;
- on sèche le mélange obtenu ;
- on calcine le produit séché.

**18/** Catalyseur dont la phase active est du type de la composition de matière selon l'une quelconque des revendications 1 à 15.

**19/** Catalyseur selon la revendication précédente, caractérisé en ce qu'il comprend un support sous forme de monolithe ou de particules.

**20/** Procédé de préparation d'acides carboxyliques insaturés, en phase gaz, caractérisé en ce que l'on effectue la réaction d'un mélange gazeux comprenant un alcane et une source d'oxygène, en présence d'un catalyseur selon l'une des revendications 18 ou 19.

**21/** Procédé de préparation d'acides carboxyliques insaturés, en phase gaz, caractérisé en ce que l'on effectue la réaction d'un mélange gazeux comprenant un alcane et une source d'oxygène, en présence d'un catalyseur dont la phase active comprend de l'étain, de l'oxygène et au moins un élément choisi parmi le vanadium, le niobium, le tantale, le chrome, le molybdène, le tungstène, l'antimoine, le bismuth, le tellure, le sélénium, le phosphore, l'arsenic, le germanium, l'aluminium et le silicium.

**22/** Procédé selon la revendication 21, caractérisé en ce que l'on utilise un catalyseur dont la phase active est à base d'étain, d'oxygène et d'au moins un élément choisi parmi le vanadium, le niobium, le chrome, le molybdène, le tungstène, l'antimoine, le gallium et le bismuth.

**23/** Procédé selon l'une quelconque des revendications 21 ou 22, caractérisé en ce que l'on utilise un catalyseur dont la phase active présente une composition telle que le rapport molaire étain à la somme des éléments précités est compris entre 0,1 et 10 et de préférence entre 0,5 et 2.

**24/** Procédé selon l'une quelconque des revendications 20 à 23, caractérisé en ce que l'on effectue la réaction avec un alcane acyclique comprenant 3 ou 4 atomes de carbone.

**25/** Procédé selon la revendication précédente, caractérisé en ce que l'on effectue ladite réaction en présence de propane.

**26/** Procédé selon l'une quelconque des revendications 20 à 25, caractérisé en ce que l'on utilise un alcane sous forme pure ou diluée dans un gaz inerte choisi parmi les gaz rares comme l'argon ou l'hélium, ou l'azote.

**27/** Procédé selon l'une quelconque des revendications 20 à 26, caractérisé en ce que l'on utilise comme source d'oxygène de l'air ou de l'oxygène.

**28/** Procédé selon l'une quelconque des revendications 20 à 27, caractérisé en ce que l'on effectue la réaction en présence d'eau.

**29/** Procédé selon l'une quelconque des revendications 20 à 28, caractérisé en ce que l'on effectue la réaction avec un mélange gazeux dont la composition est en dehors du domaine d'explosivité dudit mélange.

**30/** Procédé selon l'une quelconque des revendications 20 à 29, caractérisé en ce que l'on effectue la réaction avec une teneur en alcane comprise entre 10 et 70 % en volume, plus particulièrement d'au moins 20 % et de préférence d'au moins 30 %.

**31/** Procédé selon l'une quelconque des revendications 20 à 30, caractérisé en ce que l'on effectue la réaction avec une teneur en oxygène, comprise entre 10 et 70 %.

**32/** Procédé selon l'une quelconque des revendications 20 à 31, caractérisé en ce que l'on effectue la réaction avec une teneur en eau comprise entre 0 et 50 % et de préférence entre 0 et 30 %.

**33/** Procédé selon l'une quelconque des revendications 20 à 32 caractérisé en ce que l'on effectue la réaction avec une teneur en gaz diluant comprise entre 0 et 50 % et de préférence entre 0 et 30 %.

**34/** Procédé selon l'une quelconque des revendications 20 à 33, caractérisé en ce que l'on effectue la réaction à une température comprise entre 250 et 600°C et de préférence entre 300 et 500°C.
